(19) Europäisches Patentamt / European Patent Office / Office européen des brevets

(11) **EP 4 653 871 A1**

(12) **EUROPEAN PATENT APPLICATION**

(43) Date of publication:
26.11.2025 Bulletin 2025/48

(21) Application number: 25178501.0

(22) Date of filing: 23.05.2025

(51) International Patent Classification (IPC):
*G01N 33/86* *(2006.01)*

(52) Cooperative Patent Classification (CPC):
**G01N 33/86;** G01N 2800/224

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 24.05.2024 US 202463651747 P

(71) Applicant: Instrumentation Laboratory Company
Bedford, MA 01730 (US)

(72) Inventors:
• **CHEN, Xiaohong**
**Bedford 01730 (US)**
• **BOTTENUS, Ralph**
**Bedford 01730 (US)**

(74) Representative: **Müller-Boré & Partner**
**Patentanwälte PartG mbB**
**Friedenheimer Brücke 21**
**80639 München (DE)**

(54) **COMPOSITIONS FOR DETECTION OF HYPERFIBRINOLYSIS AND FIBRINOLYTIC SHUTDOWN AND USES THEREOF**

(57) The present disclosure provides compositions for use in a hemostasis blood test, as well as related methods, systems, kits, and sample holders. The compositions include any one or more of (a) a plurality of phospholipids, (b) a plurality of activators, (c) a blood coagulation initiator, and (d) plasmin or a precursor thereof. In some embodiments, the compositions further include one or more of (e) a buffering agent, (f) a sugar, (g) a protein concentration reference standard, and (h) $CaCl_2$.

## Description

### CROSS-REFERENCE TO RELATED APPLICATIONS

This application claims the benefit of the earlier filing date of U.S. Provisional Patent Application Ser. No. 63/651,747, filed on May 24, 2024, and titled "Compositions for Detection of Hyperfibrinolysis and Fibrinolytic Shutdown and Uses Thereof," which is incorporated herein by reference in its entirety.

### BACKGROUND

Fibrinolysis activation occurs almost universally after severe trauma. Systemic hyperfibrinolysis is a key component of Acute Traumatic Coagulopathy (ATC) and is associated with poor clinical outcomes, although controversy exists over optimal treatment strategies. Furthermore, therapeutic triggers are compounded by the lack of a sensitive and rapid diagnostic tool, with discrepancy between hyperfibrinolysis diagnosis by viscoelastic hemostatic assays as compared to diagnosis by biomarkers for fibrinolysis. Current diagnostic methods appear capable of detecting the severest forms of hyperfibrinolysis, but are relatively insensitive to moderate, yet clinically significant fibrinolytic activation. Rapid diagnosis of hyperfibrinolysis would help provide timely interventions, guide dosing of antifibrinolytic drugs when treating bleeding in trauma patients, and reduce mortality rate. Thus, there is a critical need for technology for rapid and accurate diagnosis of hyperfibrinolysis and/or fibrinolysis.

### SUMMARY

The present disclosure provides compositions, methods, and systems for rapid detection of hyperfibrinolysis and fibrinolytic shutdown in a blood sample (e.g., whole blood or plasma).

Provided herein are compositions for use in a hemostasis blood test that include one or more, e.g., two, three, or all four, of (a) phospholipid components; (b) surface activators; (c) blood coagulation initiators, e.g., tissue factor; and/or (d) plasmin or a precursor thereof. In some embodiments, the compositions for use in a hemostasis blood test include one or more of each of: (a) a phospholipid component; (b) a surface activator; (c) a blood coagulation initiator, e.g., tissue factor; and (d) plasmin or a precursor thereof. In some embodiments, a composition can further include one or more of: (e) a buffering agent; (f) a stabilizing agent; (g) a protein concentration reference standard; and (h) a salt, such as a calcium salt, e.g., $CaCl_2$.

In some embodiments, the phospholipid component is present in a concentration of about 0.001 mg/mL to about 1.0 mg/mL. In some embodiments, the phospholipid component comprises a plurality of phospholipids. In some embodiments, the surface activator is present in a concentration of about 0.01% to about 1.0%. In some embodiments, the blood coagulation initiator comprises tissue factor and the tissue factor is present in a concentration of about 0.000001 mg/mL to about 0.001 mg/mL. In some embodiments, the plasmin or precursor thereof is present in a concentration of about 2.0 $\mu$g/mL to about 24 $\mu$g/mL. In some embodiments, the surface activator comprises silica particles.

Also provided herein are methods of detecting a hemostasis disorder in a subject, the method including (a) obtaining a blood sample from the subject; (b) contacting at least a portion of the blood sample to an assay reagent, wherein the assay reagent comprises any one of the compositions described herein, thereby generating an assay sample; (c) performing a blood coagulation assay on the assay sample and measuring a time parameter of the blood coagulation assay; and (d) comparing the time parameter to a reference clotting time, wherein a higher time parameter compared to the reference clotting time indicates that the subject has a hemostasis disorder.

In some embodiments, the higher time parameter is at least one or more seconds higher than the reference clotting time. In some embodiments, a lower time parameter compared to the reference clotting time indicates that the subject does not have hyperfibrinolysis or fibrinolytic shut down.

In some embodiments, the assay reagent is disposed in a sample holder. In some embodiments, the assay reagent is a dry reagent. In some embodiments, the blood coagulation assay comprises a microfluidic device-based assay.

In some embodiments, the method further comprising treating the subject, based on the subject being determined to have a hemostasis disorder, such as hyperfibrinolysis. For example, the subject can be treated by administering empiric tranexamic acid, epsilon-aminocaproic acid, or other lysine analogs, or a blood transfusion can be provided.

Also provided herein are sample holders for use in a blood coagulation assay, the sample holder including an assay reagent comprising any one of the compositions described herein.

In some embodiments, the sample holder comprises a cartridge or a cuvette. In some embodiments, the assay reagent is provided in a dried form in the sample holder. In some embodiments, the blood coagulation assay detects a hemostasis disorder in a blood sample. In some embodiments, the blood sample is a whole blood sample.

In some embodiments, the sample holder includes a first reagent that includes (a) a phospholipid component; (b) a surface activator; (c) a blood coagulation initiator, e.g., tissue factor; and (d) plasmin or a precursor thereof; and a second reagent (e.g., a control) including (a) a phospholipid component; (b) a surface activator; and (c) a blood coagulation initiator, e.g., tissue factor, without any plasmin or plasmin precursor.

Also provided herein are systems or kits for detecting a hemostasis disorder in a blood sample, the system or kit including any one of the sample holders described herein.

Also provided herein are kits including a first reagent that includes (a) a phospholipid component; (b) a surface activator; (c) a blood coagulation initiator, e.g., tissue factor; and (d) plasmin or a precursor thereof; and a second reagent (e.g., a control) including (a) a phospholipid component; (b) a surface activator; and (c) a blood coagulation initiator, e.g., tissue factor, without plasmin. Also provided herein are cartridges including a first reagent that includes (a) a phospholipid component; (b) a surface activator; (c) a blood coagulation initiator, e.g., tissue factor; and (d) plasmin or a precursor thereof; and a second reagent (e.g., a control) including (a) a phospholipid component; (b) a surface activator; and (c) a blood coagulation initiator, e.g., tissue factor, without plasmin.

As used in the specification and the appended claims, the singular forms "a," "an" and "the" include plural referents unless the context clearly dictates otherwise. It will be further understood that the terms "comprise" and/or "comprising," or "include" and/or "including," when used in this specification, specify the presence of stated features, integers, steps, operations, elements, and/or components, but do not preclude the presence or addition of one or more other features, integers, steps, operations, elements, components, an/or groups thereof. As used herein, the term "and/or" includes any and all combinations of one or more of the associated listed items.

As used herein, the term "about," when used herein in reference to a value, refers to a value that is similar in context to the referenced value. In general, those skilled in the art, familiar with the context, will appreciate the relevant degree of variance is $\pm$ 10%, in the proper context. Unless otherwise clear from the context, all numerical values provided herein are modified by the term "about."

As used herein, the term "subject" refers an organism, typically a mammal (e.g., a human). In some embodiments, a subject is suffering from a relevant disease, disorder, or condition. In some embodiments, a subject is susceptible to a disease, disorder, or condition. In some embodiments, a subject displays one or more signs or symptoms or characteristics of a disease, disorder, or condition. In some embodiments, a subject does not display any symptom or characteristic of a disease, disorder, or condition. In some embodiments, a subject is someone with one or more features characteristic of susceptibility to or risk of a disease, disorder, or condition. In some embodiments, a subject is a patient. In some embodiments, a subject is an individual to whom diagnosis and/or therapy is and/or has been administered. In some embodiments, a subject is a human.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

## BRIEF DESCRIPTION OF DRAWINGS

These and other features and advantages of the present invention will be more fully understood by reference to the following detailed description in conjunction with the attached drawings.

FIG. 1 is a graph showing results from a first assay, wherein an assay reagent included phospholipid, silica, and tissue factor. The assay was performed on a GEM Hemochron® 100 Whole Blood Hemostasis system (supplied by Instrumentation Laboratory Company (d/b/a Werfen®, Bedford, Mass. ("Werfen")).

FIG. 2 is a graph showing results from a second assay, wherein an assay reagent included silica and tissue factor. The assay was performed on a GEM Hemochron®100 instrument.

## DETAILED DESCRIPTION

While various embodiments of the invention have been shown and described herein, those skilled in the art will understand that such embodiments are provided by way of example only. Numerous variations, changes, and substitutions may occur to those skilled in the art without departing from the invention. It may be understood that various alternatives to the embodiments of the invention described area may be employed.

Acute Traumatic Coagulopathy (ATC) is a response to trauma or severe trauma in which normal blood clotting is disrupted. ATC may be characterized by impaired thrombin generation, dysfunctional platelets, increased anticoagulant activity, and dysregulation of fibrinolysis. Dysregulation of fibrinolysis, *i.e.,* deviation from physiologic fibrinolysis, may take the form of hyperfibrinolysis or fibrinolytic shutdown. Hyperfibrinolysis, which is the excessive breakdown of fibrin clots due to overactivation of the fibrinolytic system, including overactivation of the enzyme plasmin, is a key component of ATC. Hyperfibrinolysis may lead to hemorrhage and uncontrolled bleeding. Conversely, in fibrinolytic shutdown, fibrinolysis is severely suppressed or absent, causing clots to persist or grow unchecked. Fibrinolytic shutdown (or hypofibrinolysis) may lead to organ ischemia, multi-organ failure, and death. In addition to occurring following trauma, hyperfibrinolysis and/or fibrinolytic shutdown may be observed during or following obstetrical hemorrhage, liver transplant, or exogenous tissue plasminogen activator (tPA) administration.

Hyperfibrinolysis and fibrinolytic shutdown are each associated with poor clinical outcomes. There is uncertainty about optimal treatment strategies. Additionally, a decision regarding when and what care to initiate for a patient, such as a determination that a therapeutic trigger has been met, may be complicated by the lack of a sensitive and rapid diagnostic tool. For example, there may be discrepancies between hyperfibrinolysis diagnosis by viscoelastic hemostatic assays as compared to diagnosis by biomarkers for fibrinolysis. Current diagnostic methods appear capable of detecting the severest forms of hyperfibrinolysis, but are relatively insensitive to moderate, yet clinically significant fibrinolytic activation. Rapid diagnosis of hyperfibrinolysis would help provide timely interventions and guide dosing of antifibrinolytic drugs when treating bleeding in trauma patients, reducing mortality rate. Thus, there is a critical need for improved diagnosis, including more rapid and accurate diagnosis, of hyperfibrinolysis and/or fibrinolysis.

Uncontrolled massive bleeding with subsequent derangement of the coagulation system is a major challenge in the management of both surgical and seriously injured patients. Under physiological conditions, activators and inhibitors of coagulation regulate the sensitive balance between clot formation and fibrinolysis. In some cases, excessive and diffuse bleeding is caused by systemic activation of fibrinolysis, i.e., hyperfibrinolysis (HF). Uncontrolled HF is associated with a high mortality. Conversely, the shutdown of fibrinolysis has also been observed as a pathologic phenomenon, which can lead to organ failure and death in many injured and/or critically ill patients. There is a growing need for timely and reliable hemostasis blood testing (e.g., conventional coagulation tests (CCT) or viscoelastic testing (VET)) that can examine the process of clot formation and fibrinolysis in whole blood and plasma products.

However, a major limitation of current technologies is that they typically require 60-90 minutes before a result on fibrinolysis activity is obtained, and the magnitude of the differences between normal and hyperfibrinolytic patients, as well as fibrinolytic shutdown patients, using the current assays are often very small, but clinically significant. Accordingly, improved compositions and methods are needed.

Provided herein are compositions for use in a hemostasis blood test that include (a) optionally, a phospholipid component (e.g., one or more synthetic phospholipids), (b) optionally, a surface activator (e.g., silica particles), (c) a blood coagulation initiator (e.g., Tissue Factor, such as recombinant human tissue factor (RTF)); and (d) plasmin or a precursor thereof (e.g., plasminogen). In some embodiments, the composition can further include one or more of (e) a buffering agent (e.g., HEPES buffer), (f) a stabilizing agent, such as one or more different sugars (e.g., trehalose), (g) a protein concentration reference standard (e.g., bovine serum albumin (BSA)), and (h) $CaCl_2$.

Also provided herein are methods, systems, and kits for detecting hyperfibrinolysis or fibrinolytic shut down in a subject that use any one of the compositions described herein.

Various non-limiting aspects of such compositions are described herein and can be used in any combination without limitation. Additional aspects of various components of methods of making and using the compositions are known in the art.

**Compositions for Detection of a Hemostasis Disorder**

Hemostasis is a process that leads to cessation of bleeding from a blood vessel. Hemostasis is vital to human health as a balanced and tightly regulated process that relies on an intricate balance between coagulation and fibrinolysis, wherein fibrinolysis is a physiologic process that maintains microvascular patency by breaking down excessive fibrin clots.

Hyperfibrinolysis, which is a coagulation disorder of excessive clot degradation, is associated with a doubling of mortality in injury patients. Hyperfibrinolysis is the state of excessive activation of the fibrinolytic pathway, wherein the excessive activation of plasmin, or impaired clearance or inactivation, can result in excessive proteolysis of both fibrin and fibrinogen. In some embodiments, hyperfibrinolysis is observed in trauma, obstetrical hemorrhage, during liver transplant, and following exogenous tissue plasminogen activator (tPA) administration.

Conversely, fibrinolytic shutdown is an acute impairment of fibrinolysis. Fibrinolytic shutdown is common in later phases following trauma and is also associated with increased mortality. In some embodiments, fibrinolytic shutdown can result in multisystem organ failure related to hypercoagulability and/or microvascular occlusion.

In some embodiments, hemostasis blood tests can be used to evaluate whole blood hemostasis by assessing specific components (e.g., plasma components) and/or portions (e.g., coagulation) of the hemostasis process. In some embodiments, a hemostasis blood test can include a conventional coagulation test CCT, including the GEM® Hemochron® test, that measures the time necessary for blood to clot. In some embodiments, a traditional coagulation assay can include prothrombin time (PT)/international normalized ratio (INR), activated partial thromboplastin time (aPTT), fibrinogen, and platelets (PLTs). In some embodiments, a hemostasis blood test can comprise viscoelastic testing (VET), including rotational thromboelastometry (ROTEM), thrombelastography (TEG), and sonic estimation of elasticity via resonance sonorheometry.

However, conventional hemostasis assays can typically require about 60-90 minutes before a result indicative of fibrinolysis activity is obtained. In some cases, tranexamic acid is an antifibrinolytic agent that is administered to control bleeding and slowing down the breakdown of blood clots. However, in trauma patients, tranexamic acid may be administered blindly, because for the tranexamic acid to be effective, it needs to be administered within 3 hours of injury.

The present disclosure describes compositions, methods, systems, and kits that can reduce turnaround times for the conventional hemostasis assays.

Provided herein are compositions for use in a hemostasis blood test that include (a) one or a plurality of different phospholipids (e.g., synthetic phospholipids), (b) one or a plurality of different surface activators (e.g., silica particles), (c) a blood coagulation initiator (e.g., tissue factor, such as recombinant tissue factor); and (d) plasmin or a precursor thereof (e.g., plasminogen). In some embodiments, the composition can further include one or more of (e) a buffering agent (e.g., HEPES buffer), (f) a stabilizing agent (e.g., trehalose), (g) a protein concentration reference standard (e.g., bovine serum albumin (BSA)), and (h) $CaCl_2$.

## Phospholipids

The compositions described herein may include a phospholipid component which may include one type of, or more than one different types of, phospholipids. In some embodiments, the one of the plurality of phospholipids is from a natural source. In some embodiments, the one of the plurality of phospholipids includes synthetic phospholipids. For example, the phospholipids may be selected from one or more of glycerophospholipids (such as phosphatidylcholine, phosphatidylethanolamine, phosphatidylserine, phosphatidylinositol, phosphatidic acid, and cardiolipin), plasmalogens (such as plasmenylcholine and plasmenylethanolamine), lysophosphatidylcholine, lysophosphatidic acid, and sphingomyelin.

In some embodiments, the composition includes the one of the plurality of phospholipids at a concentration of about 0.001 mg/mL to about 1.0 mg/mL (e.g., about 0.005 mg/mL to about 1.0 mg/mL, about 0.01 mg/mL to about 1.0 mg/mL, about 0.05 mg/mL to about 1.0 mg/mL, about 0.1 mg/mL to about 1.0 mg/mL, about 0.25 mg/mL to about 1.0 mg/mL, about 0.5 mg/mL to about 1.0 mg/mL, about 0.75 mg/mL to about 1.0 mg/mL, about 0.001 mg/mL to about 0.75 mg/mL, about 0.005 mg/mL to about 0.75 mg/mL, about 0.01 mg/mL to about 0.75 mg/mL, about 0.05 mg/mL to about 0.75 mg/mL, about 0.1 mg/mL to about 0.75 mg/mL, about 0.25 mg/mL to about 0.75 mg/mL, about 0.5 mg/mL to about 0.75 mg/mL, about 0.001 mg/mL to about 0.5 mg/mL, about 0.005 mg/mL to about 0.5 mg/mL, about 0.01 mg/mL to about 0.5 mg/mL, about 0.05 mg/mL to about 0.5 mg/mL, about 0.1 mg/mL to about 0.5 mg/mL, about 0.25 mg/mL to about 0.5 mg/mL, about 0.001 mg/mL to about 0.25 mg/mL, about 0.005 mg/mL to about 0.25 mg/mL, about 0.01 mg/mL to about 0.25 mg/mL, about 0.05 mg/mL to about 0.25 mg/mL, about 0.1 mg/mL to about 0.25 mg/mL, about 0.001 mg/mL to about 0.1 mg/mL, about 0.005 mg/mL to about 0.1 mg/mL, about 0.01 mg/mL to about 0.1 mg/mL, about 0.05 mg/mL to about 0.1 mg/mL, about 0.001 mg/mL to about 0.05 mg/mL, about 0.005 mg/mL to about 0.05 mg/mL, about 0.01 mg/mL to about 0.05 mg/mL, about 0.001 mg/mL to about 0.01 mg/mL, about 0.005 mg/mL to about 0.01 mg/mL, or about 0.001 mg/mL to about 0.0005 mg/mL).

## Surface Activators

The compositions described herein may also include a surface activator, or a plurality of different types of surface activators. Surface activators includes particles or molecules that promote blood coagulation. For example, surface activators may promote blood coagulation by providing a platform that facilitates the assembly and activation of clotting factors, particularly within the intrinsic pathway. For example, blood coming into contact with a surface activator may facilitate activation of Factor XII (Hageman factor) to Factor XIIa, part of the cascade activating Factors XI and IX and ultimately leading to thrombin generation and fibrin formation.

Examples of surface activators include, but are not limited to, glass, silica, kaolin, bentonite, diatomaceous earth, thrombin, snake venoms, and ellagic acid. In some embodiments, a plurality of surface activators comprises silica particles. In some embodiments, the surface activator comprises a silica dispersion. In an example, the silica may be a fumed silica formulation, such as the fumed silica formulation available from the Cabot Corporation (Boston, Mass.) under the tradename CAB-O-SPERSE® 1030K Aqueous Dispersion, having approximately 30% solids.

In some embodiments, the composition includes a surface activator, wherein the surface activator includes silica particles at a concentration of about 0.01% (w/w) to about 1.0% (w/w) (e.g., about 0.01% (w/w) to about 0.75% (w/w), about 0.01% (w/w) to about 0.5% (w/w), about 0.01% (w/w) to about 0.25% (w/w), about 0.01% (w/w) to about 0.1% (w/w), about 0.01% (w/w) to about 0.05% (w/w), about 0.05% (w/w) to about 1.0% (w/w), about 0.05% (w/w) to about 0.75% (w/w), about 0.05% (w/w) to about 0.5% (w/w), about 0.05% (w/w) to about 0.25% (w/w), about 0.05% (w/w) to about 0.1% (w/w), about 0.1% (w/w) to about 1.0% (w/w), about 0.1% (w/w) to about 0.75% (w/w), about 0.1% (w/w) to about 0.5% (w/w), about 0.1% (w/w) to about 0.25% (w/w), about 0.25% (w/w) to about 1.0% (w/w), about 0.25% (w/w) to about 0.75% (w/w), about 0.25% (w/w) to about 0.5% (w/w), about 0.5% (w/w) to about 1.0% (w/w), about 0.5% (w/w) to about 0.75% (w/w), or about 0.75% (w/w) to about 1.0% (w/w)).

Blood Coagulation Initiator

The compositions described herein may include a blood coagulation initiator or a plurality of different types of blood coagulation initiators. Examples of blood coagulation initiators include Tissue Factor (TF) and thromboplastin. More particularly, the blood coagulation initiator may be Tissue Factor. The blood coagulation initiator may be a recombinant human tissue factor (RTF), such as the RTF provided under the tradename RecombiPlastTin® within the HemosIL® line of reagents provided by Werfen.

Tissue factor (TF), also known as Factor III or CF142 is the primary cellular initiator of blood coagulation. Under normal physiological conditions, tissue factor is expressed by cells within subendothelial tissues (such as smooth muscle and fibroblasts) and is not exposed to circulating blood. When vascular injury occurs, TF becomes exposed to the bloodstream and binds with, e.g., circulating Factor VII, forming the TF-Factor VIIa complex. This complex activates Factors IX and X, triggering a cascade that leads to thrombin generation and ultimately the formation of a stable fibrin clot.

In some embodiments, a blood coagulation initiator comprises a blood coagulation initiator from a natural source. In some embodiments, a blood coagulation initiator comprises a blood coagulation initiator from a mammal (e.g., a rabbit). In some embodiments, a blood coagulation initiator comprises a synthetic blood coagulation initiator. In some embodiments, a blood coagulation initiator comprises a recombinant blood coagulation initiator.

In some embodiments, the composition includes a blood coagulation initiator at a concentration of about 0.000001 mg/mL to about 0.001 mg/mL (e.g., about 0.000005 mg/mL to about 0.001 mg/mL, about 0.00001 mg/mL to about 0.001 mg/mL, about 0.00005 mg/mL to about 0.001 mg/mL, about 0.0001 mg/mL to about 0.001 mg/mL, about 0.0005 mg/mL to about 0.001 mg/mL, about 0.000001 mg/mL to about 0.0005 mg/mL, about 0.000005 mg/mL to about 0.0005 mg/mL, about 0.00001 mg/mL to about 0.0005 mg/mL, about 0.00005 mg/mL to about 0.0005 mg/mL, about 0.0001 mg/mL to about 0.0005 mg/mL, about 0.000001 mg/mL to about 0.0001 mg/mL, about 0.000005 mg/mL to about 0.0001 mg/mL, about 0.00001 mg/mL to about 0.0001 mg/mL, about 0.00005 mg/mL to about 0.0001 mg/mL, about 0.000001 mg/mL to about 0.00005 mg/mL, about 0.000005 mg/mL to about 0.00005 mg/mL, about 0.00001 mg/mL to about 0.00005 mg/mL, about 0.000001 mg/mL to about 0.00001 mg/mL, about 0.000005 mg/mL to about 0.00001 mg/mL, or about 0.000001 mg/mL to about 0.000005 mg/mL).

Plasmin

The compositions described herein may include plasmin or a precursor thereof. In some embodiments, the composition includes plasmin or plasminogen. Plasmin is an enzyme that degrades fibrin into fibrin degradation products during fibrinolysis, and plasminogen is an inactive precursor of plasmin that is enzymatically converted to plasmin by a plasminogen activator (e.g., a tissue plasminogen activator (tPA) and urokinase). In some implementations, the compositions include plasminogen and a plasminogen activator. In some embodiments, the composition includes plasmin at a concentration of about 2.0 $\mu$g/mL to about 24 $\mu$g/mL (e.g., about 2.0 $\mu$g/mL to about 20 $\mu$g/mL, about 2.0 $\mu$g/mL to about 15 $\mu$g/mL, about 2.0 $\mu$g/mL to about 10 $\mu$g/mL, about 2.0 $\mu$g/mL to about 8.0 $\mu$g/mL, about 2.0 $\mu$g/mL to about 6.0 $\mu$g/mL, about 2.0 $\mu$g/mL to about 4.0 $\mu$g/mL, about 4.0 $\mu$g/mL to about 24 $\mu$g/mL, about 4.0 $\mu$g/mL to about 20 $\mu$g/mL, about 4.0 $\mu$g/mL to about 15 $\mu$g/mL, about 4.0 $\mu$g/mL to about 10 $\mu$g/mL, about 4.0 $\mu$g/mL to about 8.0 $\mu$g/mL, about 4.0 $\mu$g/mL to about 6.0 $\mu$g/mL, about 6.0 $\mu$g/mL to about 24 $\mu$g/mL, about 6.0 $\mu$g/mL to about 20 $\mu$g/mL, about 6.0 $\mu$g/mL to about 15 $\mu$g/mL, about 6.0 $\mu$g/mL to about 10 $\mu$g/mL, about 6.0 $\mu$g/mL to about 8.0 $\mu$g/mL, about 8.0 $\mu$g/mL to about 24 $\mu$g/mL, about 8.0 $\mu$g/mL to about 20 $\mu$g/mL, about 8.0 $\mu$g/mL to about 15 $\mu$g/mL, about 8.0 $\mu$g/mL to about 10 $\mu$g/mL, about 10 $\mu$g/mL to about 24 $\mu$g/mL, about 10 $\mu$g/mL to about 20 $\mu$g/mL, about 10 $\mu$g/mL to about 15 $\mu$g/mL, about 15 $\mu$g/mL to about 24 $\mu$g/mL, about 15 $\mu$g/mL to about 20 $\mu$g/mL, or about 20 $\mu$g/mL to about 24 $\mu$g/mL).

Buffering Agent

In some embodiments, the compositions described herein can further include a buffering agent. In some embodiments, a buffering agent comprises a Good's buffer, wherein the Good's buffer can include MES, Bis-Tris, ADA, PIPES, ACES, MOPSO, BES, MOPS, TES, HEPES, DIPSO, TAPSO, POPSO, HEPPSO, EPPS, Tricine, Bicine, TAPS, CHES, citric acid, phosphoric acid, acetic acid, imidazole, barbital, GTA, or any combinations thereof. In some embodiments, a buffering agent comprises a HEPES (4-(2-hydroxyethyl)-1-piperazineethanesulfonic acid) buffer.

In some embodiments, the composition includes a buffering agent at a concentration of about 1 mM to about 500 mM (e.g., about 5 mM to about 500 mM, about 10 mM to about 500 mM, about 20 mM to about 500 mM, about 40 mM to about 500 mM, about 60 mM to about 500 mM, about 80 mM to about 500 mM, about 100 mM to about 500 mM, about 120 mM to about 500 mM, about 140 mM to about 500 mM, about 160 mM to about 500 mM, about 180 mM to about 500 mM, about 200 mM to about 500 mM, about 250 mM to about 500 mM, about 300 mM to about 500 mM, about 350 mM to about 500 mM, about 400 mM to about 500 mM, about 450 mM to about 500 mM, about 1 mM to about 450 mM, about 5 mM to about 450 mM, about 10 mM to about 450 mM, about 20 mM to about 450 mM, about 40 mM to about 450 mM, about 60 mM to about 450 mM, about 80 mM to about 450 mM, about 100 mM to about 450 mM, about 120 mM to about 450 mM, about 140 mM to about 450 mM, about 160 mM to about 450 mM, about 180 mM to about 450 mM, about 200 mM to about 450 mM, about 250 mM to about 450 mM, about 300 mM to about 450 mM, about 350 mM to about 450 mM, about 400 mM to about 450 mM, about 1 mM to about 400 mM, about 5 mM to about 400 mM, about 10 mM to about 400 mM, about 20 mM to about 400 mM, about 40 mM to about 400 mM, about 60 mM to about 400 mM, about 80 mM to about 400 mM, about 100 mM to about 400 mM, about 120 mM to about 400 mM, about 140 mM to about 400 mM, about 160 mM to about 400 mM, about 180 mM to about 400 mM, about 200 mM to about 400 mM, about 250 mM to about 400 mM, about 300 mM to about 400 mM, about 350 mM to about 400 mM, about 1 mM to about 350 mM, about 5 mM to about 350 mM, about 10 mM to about 350 mM, about 20 mM to about 350 mM, about 40 mM to about 350 mM, about 60 mM to about 350 mM, about 80 mM to about 350 mM, about 100 mM to about 350 mM, about 120 mM to about 350 mM, about 140 mM to about 350 mM, about 160 mM to about 350 mM, about 180 mM to about 350 mM, about 200 mM to about 350 mM, about 250 mM to about 350 mM, about 300 mM to about 350 mM, about 1 mM to about 300 mM, about 5 mM to about 300 mM, about 10 mM to about 300 mM, about 20 mM to about 300 mM, about 40 mM to about 300 mM, about 60 mM to about 300 mM, about 80 mM to about 300 mM, about 100 mM to about 300 mM, about 120 mM to about 300 mM, about 140 mM to about 300 mM, about 160 mM to about 300 mM, about 180 mM to about 300 mM, about 200 mM to about 300 mM, about 250 mM to about 300 mM, about 1 mM to about 250 mM, about 5 mM to about 250 mM, about 10 mM to about 250 mM, about 20 mM to about 250 mM, about 40 mM to about 250 mM, about 60 mM to about 250 mM, about 80 mM to about 250 mM, about 100 mM to about 250 mM, about 120 mM to about 250 mM, about 140 mM to about 250 mM, about 160 mM to about 250 mM, about 180 mM to about 250 mM, about 200 mM to about 250 mM, about 1 mM to about 200 mM, about 5 mM to about 200 mM, about 10 mM to about 200 mM, about 20 mM to about 200 mM, about 40 mM to about 200 mM, about 60 mM to about 200 mM, about 80 mM to about 200 mM, about 100 mM to about 200 mM, about 120 mM to about 200 mM, about 140 mM to about 200 mM, about 160 mM to about 200 mM, about 180 mM to about 200 mM, about 1 mM to about 180 mM, about 5 mM to about 180 mM, about 10 mM to about 180 mM, about 20 mM to about 180 mM, about 40 mM to about 180 mM, about 60 mM to about 180 mM, about 80 mM to about 180 mM, about 100 mM to about 180 mM, about 120 mM to about 180 mM, about 140 mM to about 180 mM, about 160 mM to about 180 mM, about 1 mM to about 160 mM, about 5 mM to about 160 mM, about 10 mM to about 160 mM, about 20 mM to about 160 mM, about 40 mM to about 160 mM, about 60 mM to about 160 mM, about 80 mM to about 160 mM, about 100 mM to about 160 mM, about 120 mM to about 160 mM, about 140 mM to about 160 mM, about 1 mM to about 140 mM, about 5 mM to about 140 mM, about 10 mM to about 140 mM, about 20 mM to about 140 mM, about 40 mM to about 140 mM, about 60 mM to about 140 mM, about 80 mM to about 140 mM, about 100 mM to about 140 mM, about 120 mM to about 140 mM, about 1 mM to about 120 mM, about 5 mM to about 120 mM, about 10 mM to about 120 mM, about 20 mM to about 120 mM, about 40 mM to about 120 mM, about 60 mM to about 120 mM, about 80 mM to about 120 mM, about 100 mM to about 120 mM, about 1 mM to about 100 mM, about 5 mM to about 100 mM, about 10 mM to about 100 mM, about 20 mM to about 100 mM, about 40 mM to about 100 mM, about 60 mM to about 100 mM, about 80 mM to about 100 mM, about 1 mM to about 80 mM, about 5 mM to about 80 mM, about 10 mM to about 80 mM, about 20 mM to about 80 mM, about 40 mM to about 80 mM, about 60 mM to about 80 mM, about 1 mM to about 60 mM, about 5 mM to about 60 mM, about 10 mM to about 60 mM, about 20 mM to about 60 mM, about 40 mM to about 60 mM, about 1 mM to about 40 mM, about 5 mM to about 40 mM, about 10 mM to about 40 mM, about 20 mM to about 40 mM, about 1 mM to about 20 mM, about 5 mM to about 20 mM, about 10 mM to about 20 mM, about 1 mM to about 10 mM, about 5 mM to about 10 mM, or about 1 mM to about 5 mM).

In some embodiments, the buffer can have a pH between 4.0 and 9.0 (e.g., between 4.5 and 9.0, between 5.0 and 9.0, between 5.5 and 9.0, between 6.0 and 9.0, between 6.5 and 9.5, between 7.0 and 9.0, between 7.5 and 9.0, between 8.0 and 9.0, between 8.5 and 9.0, between 4.0 and 8.5, between 4.5 and 8.5, between 5.0 and 8.5, between 5.5 and 8.5, between 6.0 and 8.5, between 6.5. and 8.5, between 7.0 and 8.5, between 7.5 and 8.5, between 8.0 and 8.5, between 4.0 and 8.0, between 4.5 and 8.0, between 5.0 and 8.0, between 5.5 and 8.0, between 6.0 and 8.0, between 6.5. and 8.0, between 7.0 and 8.0, between 7.5 and 8.0, between 4.0 and 7.5, between 4.5 and 7.5, between 5.0 and 7.5, between 5.5 and 7.5, between 6.0 and 7.5, between 6.5. and 7.5, between 7.0 and 7.5, between 4.0 and 7.0, between 4.5 and 7.0, between 5.0 and 7.0, between 5.5 and 7.0, between 6.0 and 7.0, between 6.5. and 7.0, between 4.0 and 6.5, between 4.5 and 6.5, between 5.0 and 6.5, between 5.5 and 6.5, between 6.0 and 6.5, between 4.0 and 6.0, between 4.5 and 6.0, between 5.0 and 6.0, between 5.5 and 6.0, between 4.0 and 5.5, between 4.5 and 5.5, between 5.0 and 5.5, between 4.0 and 5.0, between 4.5 and 5.0, or between 4.0 and 4.5).

Stabilizing Agents

In some embodiments, the composition can further include one or more stabilizing agents. In some embodiments, the composition can further include one or more stabilizing agents, wherein a stabilizing agent can include a disaccharide. In some embodiments, the composition can further include one or more stabilizing agents, wherein a stabilizing agent can include a sugar. In some embodiments, a stabilizing agent can include glucose, alpha-D-mannopyranose, lactose, cellobiose, mannose, maltose, inositol, sucrose, inulin, fructose, or dextran. In some embodiments, the stabilizing agent is used as preservative and to protect components such as proteins during lyophilization or drying. In some embodiments, the stabilizing agent comprises trehalose. In some embodiments, a composition includes a stabilizing agent at a concentration of about 0.005 g/mL to about 1.0 g/mL (e.g., about 0.01 g/mL to about 1.0 g/mL, about 0.05 g/mL to about 1.0 g/mL, about 0.1 g/mL to about 1.0 g/mL, about 0.5 g/mL to about 1.0 g/mL, about 0.005 g/mL to about 0.5 g/mL, about 0.01 g/mL to about 0.5 g/mL, about 0.05 g/mL to about 0.5 g/mL, about 0.1 g/mL to about 0.5 g/mL, about 0.005 g/mL to about 0.1 g/mL, about 0.01 g/mL to about 0.1 g/mL, about 0.05 g/mL to about 0.1 g/mL, about 0.005 g/mL to about 0.05 g/mL, about 0.01 g/mL to about 0.05 g/mL, or about 0.0005 g/mL to about 0.01 g/mL).

Protein Concentration Reference Standard

In some embodiments, the composition can further include a protein concentration reference standard. In some embodiments, a protein concentration reference standard comprises albumin such as bovine serum albumin (BSA). In some embodiments, the protein concentration reference standard may include synthetic polymers, recombinant human albumins, and other non-animal-derived proteins or synthetic alternatives. In some embodiments, the protein concentration reference standard may include sericin or casein.

In some embodiments, the composition includes a protein concentration reference standard (such as BSA) at a concentration of about 0.0001 mg/mL to about 5.0 mg/mL (e.g., about 0.0001 mg/mL to about 2.5 mg/mL, about 0.0001 mg/mL to about 1.0 mg/mL, about 0.0001 mg/mL to about 0.5 mg/mL, about 0.0001 to about 0.1 mg/mL, about 0.0001 to about 0.05 mg/mL, about 0.0001 mg/mL to about 0.01 mg/mL, about 0.0001 mg/mL to about 0.005 mg/mL, about 0.0001 mg/mL to about 0.001 mg/mL, about 0.0001 mg/mL to about 0.0005 mg/mL, about 0.0005 mg/mL to about 5.0 mg/mL, about 0.0005 mg/mL to about 2.5 mg/mL, about 0.0005 mg/mL to about 1.0 mg/mL, about 0.0005 mg/mL to about 0.5 mg/mL, about 0.0005 to about 0.1 mg/mL, about 0.0005 to about 0.05 mg/mL, about 0.0005 mg/mL to about 0.01 mg/mL, about 0.0005 mg/mL to about 0.005 mg/mL, about 0.0005 mg/mL to about 0.001 mg/mL, about 0.001 mg/mL to about 5.0 mg/mL, about 0.001 mg/mL to about 2.5 mg/mL, about 0.001 mg/mL to about 1.0 mg/mL, about 0.001 mg/mL to about 0.5 mg/mL, about 0.001 to about 0.1 mg/mL, about 0.001 to about 0.05 mg/mL, about 0.001 mg/mL to about 0.01 mg/mL, about 0.001 mg/mL to about 0.005 mg/mL, about 0.005 mg/mL to about 5.0 mg/mL, about 0.005 mg/mL to about 2.5 mg/mL, about 0.005 mg/mL to about 1.0 mg/mL, about 0.005 mg/mL to about 0.5 mg/mL, about 0.005 to about 0.1 mg/mL, about 0.005 to about 0.05 mg/mL, about 0.005 mg/mL to about 0.01 mg/mL, about 0.01 mg/mL to about 5.0 mg/mL, about 0.01 mg/mL to about 2.5 mg/mL, about 0.01 mg/mL to about 1.0 mg/mL, about 0.01 mg/mL to about 0.5 mg/mL, about 0.01 to about 0.1 mg/mL, about 0.01 to about 0.05 mg/mL, about 0.05 mg/mL to about 5.0 mg/mL, about 0.05 mg/mL to about 2.5 mg/mL, about 0.05 mg/mL to about 1.0 mg/mL, about 0.05 mg/mL to about 0.5 mg/mL, about 0.05 to about 0.1 mg/mL, about 0.1 mg/mL to about 5.0 mg/mL, about 0.1 mg/mL to about 2.5 mg/mL, about 0.1 mg/mL to about 1.0 mg/mL, about 0.1 mg/mL to about 0.5 mg/mL, about 0.5 mg/mL to about 5.0 mg/mL, about 0.5 mg/mL to about 2.5 mg/mL, about 0.5 mg/mL to about 1.0 mg/mL, about 1.0 mg/mL to about 5.0 mg/mL, about 1.0 mg/mL to about 2.5 mg/mL, or about 2.5 mg/mL to about 5.0 mg/mL).

Salt

In some embodiments, the composition can further include a salt, e.g., a calcium salt, such as calcium chloride (CaCl$_2$) calcium gluconate, calcium citrate, calcium lactate, calcium acetate, calcium gluceptate, calcium aspartate; a magnesium salt, such as magnesium sulfate, magnesium chloride, magnesium carbonate, magnesium acetate, or magnesium orotate; or a zinc salt, such as zinc chloride, zine sulfate, zinc gluconate, since acetate, or zinc stearate ; or iron salt, such as iron chloride, iron sulfate, iron gluconate, iron fumarate, or iron citrate. In some embodiments, the composition includes the calcium salt at a concentration of about 0.0005 g/mL to about 0.05 g/mL (e.g., about 0.001 g/mL to about 0.05 g/mL, about 0.005 g/mL to about 0.05 g/mL, about 0.01 g/mL to about 0.05 g/mL, about 0.0005 g/mL to about 0.01 g/mL, about 0.001 g/mL to about 0.01 g/mL, about 0.005 g/mL to about 0.01 g/mL, about 0.0005 g/mL to about 0.005 g/mL, about 0.001 g/mL to about 0.005 g/mL, or about 0.0005 g/mL to about 0.001 g/mL).

**Methods of Detecting a Hemostasis Disorder**

Provided herein are methods of detecting a hemostasis disorder (e.g., hyperfibrinolysis or fibrinolytic shut down)

in a subject that include (a) obtaining a blood sample (e.g., whole blood or plasma) from the subject; (b) contacting at least a portion of the blood sample to an assay reagent, wherein the assay reagent comprises any one of the compositions described herein, thereby generating an assay sample; (c) measuring a time parameter in the assay sample by performing a blood coagulation assay on the assay sample; and (d) comparing the time parameter to a reference clotting time, wherein a higher time parameter compared to the reference clotting time indicates that the subject has hyperfibrinolysis or fibrinolytic shut down.

In some embodiments, an alternative method of detecting a hemostasis disorder in a subject can include (a) obtaining a blood sample (e.g., whole blood or plasma) from the subject; (b) contacting a first portion of the blood sample (e.g., whole blood or plasma) to a first assay reagent, wherein the first assay reagent comprises any of the compositions described herein but not including plasmin or plasminogen, thereby generating a first assay sample; (c) contacting a second portion of the blood sample (e.g., whole blood or plasma) to a second assay reagent, wherein the second assay reagent comprises (i) the composition from step b and (ii) a plasmin or plasminogen, thereby generating a second assay sample (d) measuring a first time parameter in the first assay sample and a second time parameter in the second assay sample by performing a blood coagulation assay on the first and second assay samples; and (e) analyzing the first and second time parameter, wherein a higher or equal second time parameter compared to the first blood time parameter indicates the subject has a hemostasis disorder. In some embodiments, this method can be implemented with two devices, a first device used for contacting the first portion of a blood sample, and a second device used for testing the second portion of the blood sample. In some other embodiments, this method can be implemented on a single device having two or more channels, a first channel used for contacting the first portion of a blood sample, and a second channel used for testing the second portion of the blood sample. A blood sample can be any blood sample, such as whole blood or plasma.

As used herein, the term "clotting time" or "blood clotting time" refers to the time until there is evidence of a clot forming in a blood sample (e.g., whole blood or plasma). As used herein the term "time parameter" refers to a clotting time or a maximum time (e.g., out of range time) for a test for determining a hemostasis disorder. As used herein, the term "reference clotting time" refers to a clotting time of normal blood with plasmin or plasminogen. In some embodiments, a reference clotting time can be an average of one or more clotting times determined from one or more different normal blood samples.

In some embodiments, the maximum time is used as the reference clotting time if no clotting time is found during a test. In some embodiments, a method includes measuring a time parameter in an assay sample and comparing the time parameter to a reference clotting time to determine whether a subject has a hemostasis disorder (e.g., hyperfibrinolysis or fibrinolytic shut down). In some embodiments, a higher time parameter compared to a reference clotting time indicates that a subject has hyperfibrinolysis or fibrinolytic shut down, wherein the higher time parameter is a time parameter that is higher when compared to the reference clotting time. In some embodiments, the higher time parameter is about 60 seconds or more. In some embodiments, the higher time parameter is about 80 seconds or more (e.g., about 100 seconds, about 120 seconds, about 3 minutes, about 5 minutes, about 10 minutes, or about 30 minutes or more). In some embodiments, the higher time parameter is at least one or more seconds higher (e.g., at least 5 or more, at least 10 or more, at least 20 or more, at least 30 or more, at least 60 or more, at least 120 or more, at least 180 or more, at least 240 or more, or at least 300 or more seconds higher) than the reference clotting time.

In some embodiments, a lower blood clotting time compared to a reference clotting time indicates that a subject does not have hyperfibrinolysis or fibrinolytic shut down, wherein the lower time parameter is a time parameter that is lower when compared to the reference clotting time. In some embodiments, the lower blood clotting time is about 50 second or less. In some embodiments, the lower blood clotting time is about 40 seconds or less (e.g., about 30 seconds, about 20 seconds, about 15 seconds, about 10 seconds, or about 5 seconds).

In some embodiments, an assay reagent that is used in any of the methods described herein can be disposed (*i.e.*, placed) in a sample holder. A sample holder includes a support device or a sample handling apparatus that is configured to receive any samples or reagents necessary to perform an assay. In some embodiments, the sample holder can include a cartridge or a cuvette (e.g., a consumable cuvette). In some embodiments, the assay reagent disposed in the sample holder is a dried reagent, wherein the assay reagent is dispensed and dried onto the sample holder.

In some embodiments, a method described herein includes performing a blood coagulation assay on an assay sample, wherein the blood coagulation assay comprises a microfluidic device-based assay. In some embodiments, the blood coagulation assay can be performed on a microfluidic device-based assay, wherein the microfluidic device is a handheld device, such as a GEM Hemochron®100 and Hemochron® Signature Elite instruments supplied by Werfen.

In some embodiments, a method of detecting hyperfibrinolysis or fibrinolytic shut down in a subject can be performed within a time duration of less than 5 minutes. In some embodiments, the time duration is less than 4 minutes. In some embodiments, the time duration is less than 3 minutes. In some embodiments, the time duration is less than 2 minutes. In some embodiments, the time duration is less than 1 minute.

In some embodiments, a method described herein can further include treating the subject, based on the subject being determined to have hyperfibrinolysis or fibrinolytic shut down. In some embodiments, the subject is identified as having hyperfibrinolysis. In some embodiments, the treating of the subject that has been identified as having hyperfibrinolysis can include administration of an antifibrinolytic agent. In some embodiments, the treating of the subject that has been identified as having hyperfibrinolysis can include administration of Cyklokapron® (tranexamic acid)(TXA) or Amicar® (aminocaproic acid). In some embodiments, the subject is identified as having fibrinolytic shut down. In some embodiments, the treating of the subject that has been identified as having fibrinolytic shut down can include administration of a sequential compression device (e.g., to an arm or leg of the subject), heparin, or Stanozlol® (synthetic testosterone) IM. In some embodiments, epsilon-aminocaproic acid or other lysine analogs can be administered, or a blood transfusion can be provided to treat these hemostasis disorders.

**Systems/Kits for Detecting a Hemostasis Disorder**

Provided herein are systems or kits for detecting a hemostasis disorder (e.g., hyperfibrinolysis or fibrinolytic shut down) in a blood sample (e.g., whole blood or plasma), the systems or kits including a sample holder comprising an assay reagent, wherein the assay reagent comprises any one of the compositions described herein.

Also provided herein are sample holders for use in a blood coagulation assay, the sample holder including an assay reagent comprising any one of the compositions described herein. In some embodiments, the sample holder comprises a cartridge or a cuvette (e.g., a consumable cuvette).

In some implementations the kit can include two or more sample holders. A first sample holder can include a first assay reagent, wherein the assay reagent comprises any one of the compositions described herein but not including plasmin or plasminogen. A second sample holder can include a second assay reagent, wherein the assay reagent comprises any one of the compositions described herein including plasmin or plasminogen.

In some embodiments, the sample holder is configured to include an assay reagent, wherein the assay reagent is a dry reagent in the sample holder. In some embodiments, the assay reagent can be lyophilized. In some embodiments, the assay reagent can be dried (e.g., dried with air flow with mild heat (e.g., lower than 70 °C), or dried in a desiccator at about room temperature or with mild heat). In some embodiments, the assay reagent can comprise a dried powder, film, or dried beads.

In some embodiments, the sample holder includes one channel including an assay reagent, wherein the assay reagent comprises any one of the compositions described herein. In some other embodiments, the sample holder includes a first channel and a second channel. The first channel includes a first assay reagent, wherein the first assay reagent comprises any one of the compositions described herein but not including plasmin or plasminogen. The second channel includes a second assay reagent, which includes the same assay reagent as in the first channel but also includes plasmin or plasminogen.

In some embodiments, this method can be implemented with two devices, a first device used for contacting the first portion of a blood sample (e.g., whole blood or plasma), and a second device used for testing the second portion of the blood sample. In some other embodiments, this method can be implemented on a single device having two or more channels, a first channel used for contacting the first portion of a blood sample, and a second channel used for testing the second portion of the blood sample.

In some embodiments, the sample holder is configured to be used in a blood coagulation assay, wherein the blood coagulation assay detects a hemostasis disorder (e.g., hyperfibrinolysis or fibrinolytic shut down) in a blood sample. In some embodiments, the blood sample is a whole blood sample. In some embodiments, the blood sample is a plasma sample. In some embodiments, the blood sample is from a human subject.

**Clotting Assay Systems**

In a non-limiting example, the clotting assays may be performed using the system described in U.S. Patent No. 10,175,225 ("Blood Testing System and Method") that issued on January 8, 2019, the contents of which are incorporated herein by reference. U.S. Patent No. 10,175,225 describes a blood testing system that include an analyzer console and one or more cartridges for performing tests, including a test to determine the amount of fibrinogen in a test sample using a liquid thrombin reagent such as those described herein.

Examples of systems providing clotting testing that may be performed using a cup and pin structure include Rotem® *sigma* and Rotem® *delta* systems provided by Werfen. The clotting characteristic(s) may be used by the associated analyzer console to implement the processes described herein for determining the amount of fibrinogen in the mixture and/or determining an amount thrombin inhibitor in the mixture.

In another non-limiting example, the clotting assays may be performed using a mechanical clot detection system, such as the GEM® Hemochron®100 Whole Blood Hemostasis system provided by Werfen and using mechanical clot detection in which a whole blood sample is introduced into a test cartridge, the system monitors the formation of a fibrin clot by detecting changes in the movement of a steel ball within the cartridge to determines a clotting time. Aspects of the operation of the mechanical clot detection system are described in U.S. Patent No. 11,242,848 ("Methods of Operating a Pump to Reduce or Eliminate Pump Backlash Errors") that issued on February 8, 2022, the contents of which are incorporated herein by reference.

In another non-limiting example, the clotting assays may be performed using the system described in U.S. Patent No. 11,366,093 ("Disposable System for Analysis of Hemostatic Function") that issued on June 21, 2022, the contents of which are incorporated herein by reference. This patent describes Hemosonics® Quantra® hemostasis analyzer provided by HemoSonics, LLC (Durham, NC).

In still another non-limiting example, the clotting assays may be performed using the TEG® 6s cartridge or the ClotPro® active-tip cartridge-based system provided by Haemonetics Corporation (Boston, Mass.). That system includes a cartridge containing one or more chambers that may hold a mixture of test sample such as blood and the liquid thrombin reagent described herein. Testing in the TEG® 6s system is performed using optical analysis in combination with resonant frequency technology and is used to determine clotting characteristic(s) based on the mixture such as TTC. More specifically, in examples using the TEG® 6s cartridge-based system, as described in Haemonetics® USFDA 501K Summary, "The TEG® 6s technology is based on a disposable cartridge containing up to 4 independent measurement cells. Each cell consists of a short vertically-oriented injection molded tube (ring). Detection of clotting in the TEG® 6s Hemostasis System is performed optically. A piezoelectric actuator vibrates the measurement cell(s) through a motion profile composed of summed sinusoids at different frequencies. The movement of the measurement cells will induce motion in the sample meniscus, which will be detected by a photodiode. The resulting motion of the meniscus is detected optically and analyzed by the instrument to calculate the resonant frequency and modulus of elasticity (stiffness) of the sample. By performing a Fast Fourier Transform (FFT) on meniscus motion data, the resonant frequencies can be determined. The analyzer detects the harmonic motion of a hanging drop of blood in response to external vibration. As the sample transitions from a liquid state to a gel-like state during clotting, the modulus of elasticity (stiffness) and therefore resonant frequency increase. A TEG® 6s hemostasis analyzer measures these variations in resonant frequency during clotting and lysis." The clotting characteristic(s) determined using the TEG® 6s technology may be used by analyzer to implement the processes described herein for determining the amount of fibrinogen in the mixture and/or determining an amount of thrombin inhibitor in the mixture.

Examples of the parameters for which test result values may be generated by these example viscoelastic test instruments include, but are not limited to, the following clot initiation parameters, clot kinetic parameters, clot firmness parameters, and clot lysis parameters.

| Category | ROTEM® and ClotPro® | TEG® | QUANTRA® |
|---|---|---|---|
| Clot initiation parameter | CT | CT, R-time | CT; CTH; CTR |
| Clot kinetics parameter | CFT; alpha angle | K-time; alpha angle | |
| Clot firmness parameter | A5; A10; A20; MCF | A5; A10; MA | CS; PCS; FCS |
| Clot lysis parameter | ML; LI30; LI45; LI60; LOT; LT | LY30; LY60; ML | CSL |

For ROTEM® and ClotPro®, CT refers to clotting time; CFT refers to clot formation time, the alpha angle refers to the angle tangent to the clotting curve at a two millimeter (mm) amplitude point, A5 refers to clot firmness amplitude five minutes after CT; A10 refers to clot firmness amplitude ten minutes after CT; A20 refers to clot firmness twenty minutes after CT; MCF refers to maximum clot firmness; ML refers to maximum lysis; LI30 refers to a lysis index thirty minutes after CT; LI45 refers to a lysis index 45 minutes after CT; LI60 refers to a lysis index 60 minutes after CT; LOT refers to lysis onset time; and LT refers to lysis time.

For TEG®, CT refers to clotting time, R-time refers to reaction time; K-time refers to kinetic time, the alpha angle refers to the speed of fibrin accumulation and polymerization and is closely related to K-time; A5 refers to clot firmness amplitude five minutes after CT; A10 refers to clot firmness amplitude ten minutes after CT; MA refers to maximum amplitude; ML refers to maximum lysis; LY30 refers to lysis 30 minutes after MA; and LY60 refers to lysis 60 minutes after MA.

For Quantra®, CT refers to clotting time without heparinase; CTH refers to clotting time with heparinase, CTR refers to clotting time ratio; CS refers to clot stiffness; PCS refers to platelet contribution to clot stiffness; FCS refers to fibrinogen contribution to clot stiffness; and CSL refers to clot stability to lysis.

The parameters may be obtained using an activated clotting time (ACT) test performed on the GEM Hemochron® 100 system.

The present disclosure may be implemented with a variety of hemostasis assays. A person of ordinary skill in the art will appreciate that there are a variety of hemostasis assays. Standard coagulation tests include D-dimer tests, prothrombin time (PT) tests, international normalized ratio (INR) blood tests, activated partial thromboplastin clotting time (aPTT) tests, thrombin time (TT) tests, diluted thrombin time (dTT) tests, calibrated direct oral anticoagulants (DOAC) tests, platelet function tests, and platelet count tests. The parameters may be obtained from one or more of these tests.

Platelet function tests include an aspirin test and a P2Y12 reactions units (PRU) test performed on the VerifyNow™ system. Platelet function tests may include ROTEM® platelet tests, multiplate tests, TEG® platelet mapping tests, and platelet function analyzer (PFA) 100/200 tests. The parameters may be obtained from one or more of these tests.

Two metrics that can be used to understand the effectiveness of the compositions and methods described herein are clot reading percentage and reading difference.

Clot reading percentage (Clot reading%) reflects the ratio of a test reading with plasmin (T) to a control reading without plasmin (C) and may be calculated using Equation 1:

$$\text{Clot reading \%} = \frac{\text{test reading with plasmin}}{\text{test reading without plasmin}} \times 100\% \qquad \text{(Eqn. 1)}$$

Reading difference reflects an absolute difference between a test reading with plasmin (T) and a control reading without plasmin (C) and may be calculated using Equation 2:

$$\text{Reading Difference} = |\text{test result reading with plasmin} -$$
$$\text{test result reading without plasmin}| \quad \text{(Eqn.2)}$$

Normal healthy blood samples are expected to produce lower clot reading on cuvettes containing plasmin (Test) versus cuvettes containing no plasmin (Control). Patient blood samples with hyperfibrinolysis or shutdown risk will produce equivalent or longer clot reading on cuvettes containing plasmin (Test) versus cuvettes containing no plasmin (Control). Accordingly, the gap between T/C (i.e, clot reading percentage) for healthy people and T/C for hyperfibrinolytic patients, is preferably maximized. The larger the gap, the likelier that T/C of healthy people and T/C of hyperfibrinolytic patients can be clearly divided statistically in clinical test with fewer false positive or false negative results. The calculation of T-C, i.e., reading difference, is an alternative presentation of this data, which presents absolute difference while T/C is for relative difference. Again, it is preferable to maximize the absolute difference to achieve improved test performance.

### EXAMPLES

The disclosure is further described in the following examples, which do not limit the scope of the disclosure described in the claims.

### *Example 1 - Reagent Including Phospholipid/Silica/Tissue Factor*

Normal healthy patient blood samples were used for screening tests. Each blood sample was tested with cuvettes containing no plasmin in parallel with cuvettes containing 3 levels of plasmin.

Citrated blood sample was tested with a cartridge containing zero plasmin and a specific level of plasmin, respectively, on a GEM Hemochron® 100 instrument to obtain a pair of test results. The ratio of the test result with plasmin versus the test result without plasmin was used to generate the results. The compositions of the reagents used in the assay are described in **Table** 1.

Table 1. Composition of the Reagents in Solution

| Component | Reagents | | | | |
|---|---|---|---|---|---|
| | Reagent 1 | Reagent 2 | Reagent 3 | Reagent 4 | Reagent 5 |
| HEPES | 0.004 g/mL | 0.004 g/mL | 0.004 g/mL | 0.004 g/mL | 0.004 g/mL |
| NaOH | 0.0084 g/mL | 0.0084 g/mL | 0.0084 g/mL | 0.0084 g/mL | 0.0084 g/mL |
| blended phospholipids | $2.9 \times 10^{-5}$ g/mL | $2.9 \times 10^{-5}$ g/mL | $2.9 \times 10^{-5}$ g/mL | $2.9 \times 10^{-5}$ g/mL | $2.9 \times 10^{-5}$ g/mL |
| Fumed silica | 0.6% (v/v) | 0.6% (v/v) | 0.6% (v/v) | 0.6% (v/v) | 0.6% (v/v) |
| RTF | 5.6% (v/v) | 5.6% (v/v) | 5.6% (v/v) | 5.6% (v/v) | 5.6% (v/v) |
| Trehalose dihydrate | 0.024 g/mL | 0.024 g/mL | 0.024 g/mL | 0.024 g/mL | 0.024 g/mL |
| BSA | $1.0 \times 10^{-6}$ g/mL | $1.0 \times 10^{-6}$ g/mL | $1.0 \times 10^{-6}$ g/mL | $1.0 \times 10^{-6}$ g/mL | $1.0 \times 10^{-6}$ g/mL |
| $CaCl_2$ dihydrate | 0.0018 g/mL | 0.0018 g/mL | 0.0018 g/mL | 0.0018 g/mL | 0.0018 g/mL |

(continued)

| Component | Reagents | | | | |
|---|---|---|---|---|---|
| | Reagent 1 | Reagent 2 | Reagent 3 | Reagent 4 | Reagent 5 |
| plasmin | 0 | 9.7 μg/mL | 11.4 μg/mL | 13.6 μg/mL | 17.0 μg/mL |

Test results were determined for each of the assays described in Table 1 using the GEM Hemochron® 100 instrument. The test results are reported in Table 2.

Table 2. Test Result Mean Value Based on Two Replicates

| Added Plasmin Conc (μg/mL) | Samples | | | | |
|---|---|---|---|---|---|
| | 2345 | 2017 | 2067 | 2224 | 2432 |
| 0.0 | 73.5 | 80.0 | 90.0 | 73.5 | 81.0 |
| 9.7 | 39.0 | 34.0 | 40.0 | 39.5 | 38.0 |
| 11.4 | 38.0 | 41.0 | 36.5 | 37.5 | 35.0 |
| 13.6 | 39.0 | 35.5 | 36.5 | 35.0 | 38.0 |
| 17.0 | ** | ** | ** | ** | ** |
| ** Indicates out-of-range-high reading | | | | | |

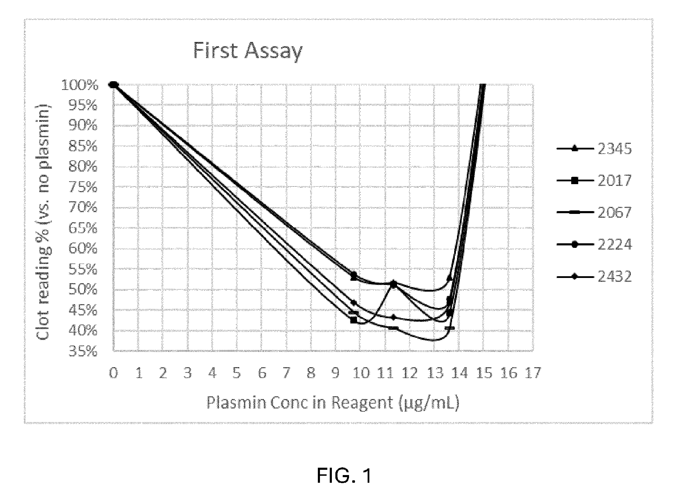

Clot reading percentage and reading difference were calculated as described above. The clot reading % results are shown in **Table 3** and in FIG. 1. The reading difference values are reported in **Table 4.**

**Table 3. Clot Reading % vs. Control Without Plasmin**

| Added Plasmin Conc (μg/mL) | Samples | | | | |
|---|---|---|---|---|---|
| | 2345 | 2017 | 2067 | 2224 | 2432 |
| 0.0 | 100.0% | 100.0% | 100.0% | 100.0% | 100.0% |
| 9.7 | 53.1% | 42.5% | 44.4% | 53.7% | 46.9% |
| 11.4 | 51.7% | 51.3% | 40.6% | 51.0% | 43.2% |
| 13.6 | 53.1% | 44.4% | 40.6% | 47.6% | 46.9% |
| 17.0* | 200.0% | 200.0% | 200.0% | 200.0% | 200.0% |
| *Data at 17.0 ug/mL used nominal data 200% to indicate high readings. The actual test data was shown as Out-of-range-high which meant data outside the measurement range. | | | | | |

**Table 4. Reading Difference (Absolute Number) with Plasmin versus Without Plasmin**

| Added Plasmin Conc (μg/mL) | Samples | | | | |
|---|---|---|---|---|---|
| | 2345 | 2017 | 2067 | 2224 | 2432 |
| 9.7 | 34.5 | 46.0 | 50.0 | 34.0 | 43.0 |
| 11.4 | 35.5 | 39.0 | 53.5 | 36.0 | 46.0 |
| 13.6 | 34.5 | 44.5 | 53.5 | 38.5 | 43.0 |
| 17.0* | N/A | N/A | N/A | N/A | N/A |

The Mean of Clot reading percentage for the three concentration levels of plasmin with quantitative results as shown in **Table 3** (9.7, 11.4 and 13.6 μg/mL) was 47%. The mean reading difference absolute number was around 42 seconds. The graph (Fig. 1) displays mean clot reading percentages of test reagent versus negative control reagent across levels of plasmin concentrations for blood samples from five different healthy donors.

*Example 2 - Reagent Including Tissue Factor*

Normal healthy patient blood samples were used for screening tests. Each blood sample was tested with cuvettes containing no plasmin in parallel with cuvettes containing three levels of plasmin.

The assays were performed according to a method described herein, wherein blood samples from three donors were tested on a GEM Hemochron®100 instrument to obtain test results. The composition of the reagents that were used in the assay are described in **Table 5.**

**Table 5. Composition of the Reagents in Solution**

| Component | Reagents | | | | |
|---|---|---|---|---|---|
| | Reagent 6 | Reagent 7 | Reagent 8 | Reagent 9 | Reagent 10 |
| HEPES | 0.004 g/mL | 0.004 g/mL | 0.004 g/mL | 0.004 g/mL | 0.004 g/mL |
| NaOH | 0.0084 g/mL | 0.0084 g/mL | 0.0084 g/mL | 0.0084 g/mL | 0.0084 g/mL |
| RTF | 4.0% (v/v) | 4.0% (v/v) | 4.0% (v/v) | 4.0% (v/v) | 4.0% (v/v) |
| Trehalose dihydrate | 0.024 g/mL | 0.024 g/mL | 0.024 g/mL | 0.024 g/mL | 0.024 g/mL |
| $CaCl_2$ dihydrate | 0.0018 g/mL | 0.0018 g/mL | 0.0018 g/mL | 0.0018 g/mL | 0.0018 g/mL |
| plasmin | 0 | 9.7 µg/mL | 11.4 µg/mL | 13.6 µg/mL | 17.0 µg/mL |

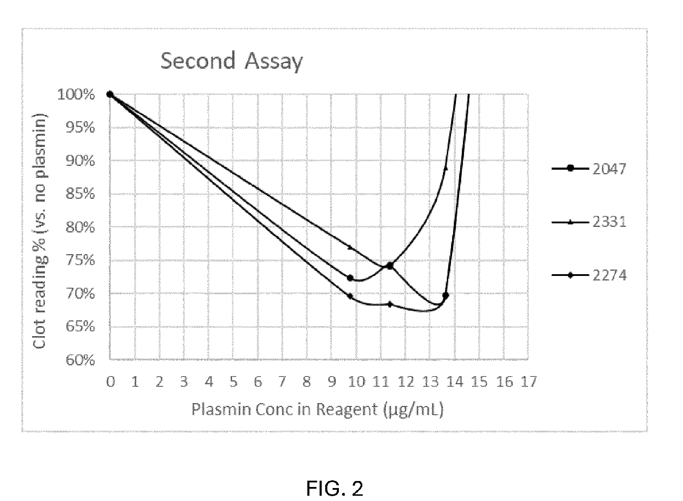

Test results were determined for each of the assays shown in **Table 6.** Clot reading % results were calculated and are shown in **Table 7** and in FIG. 2. Reading difference results were calculated and are shown in **Table 8.**

**Table 6. Test Result Mean Value Based on Three Replicates**

| Added Plasmin Conc µg/mL | Samples | | |
|---|---|---|---|
| | 2047 | 2331 | 2274 |
| 0.0 | 50.7 | 45.0 | 56.0 |
| 9.7 | 36.7 | 34.7 | 39.0 |
| 11.4 | 37.7 | 33.3 | 38.3 |
| 13.6 | 35.3 | 40.0 | 39.0 |
| 17.0 | ** | ** | ** |
| ** Indicates out-of-range-high reading | | | |

**Table 7. Clot Reading %**

| Added Plasmin Conc µg/mL | Samples | | |
|---|---|---|---|
| | 2047 | 2331 | 2274 |
| 0.0 | 100.0% | 100.0% | 100.0% |
| 9.7 | 72.4% | 77.0% | 69.6% |
| 11.4 | 74.3% | 74.1% | 68.5% |
| 13.6 | 69.7% | 88.9% | 69.6% |
| 17.0* | 200.0% | 200.0% | 200.0% |
| *Data at 17.0 ug/mL used nominal data 200% to indicate high readings. The actual test data was shown as Out-of-range-high which meant data outside the measurement range. | | | |

**Table 8. Reading Difference (Absolute Number) with Plasmin versus Without Plasmin**

| | Samples | | |
|---|---|---|---|
| Added Plasmin Conc µg/mL | 2047 | 2331 | 2274 |

(continued)

| Samples | | | |
|---|---|---|---|
| 9.7 | 14.0 | 13.0 | 15.3 |
| 11.4 | 10.3 | 11.7 | 5.0 |
| 13.6 | 17.0 | 17.7 | 17.0 |
| 17.0 | N/A | N/A | N/A |

For Example 2, the overall Mean of Clot reading percentage was 74%. The overall mean reading difference was around 13 sec.

### *Example 3 - Reagent Including Phospholipids/Tissue Factor*

Normal healthy patient blood samples were used for screening tests. Each blood sample was tested with cuvettes containing no plasmin in parallel with cuvettes containing three levels of plasmin.

The assays were performed according to a method described herein, wherein blood samples from 5 donors were tested on a GEM Hemochron®100 instrument to obtain test results. The composition of the reagents used in the assay are described in **Table 9.**

**Table 9. Composition of the Reagents in Solution**

| Component | Reagents | | | |
|---|---|---|---|---|
| | Reagent 1 | Reagent 2 | Reagent 3 | Reagent 4 |
| HEPES | 0.004 g/mL | 0.004 g/mL | 0.004 g/mL | 0.004 g/mL |
| NaOH | 0.0084 g/mL | 0.0084 g/mL | 0.0084 g/mL | 0.0084 g/mL |
| blended phospholipids | $2.9 \times 10^{-5}$ g/mL | $2.9 \times 10^{-5}$ g/mL | $2.9 \times 10^{-5}$ g/mL | $2.9 \times 10^{-5}$ g/mL |
| RTF | 5.6% (v/v) | 5.6% (v/v) | 5.6% (v/v) | 5.6% (v/v) |
| Trehalose dihydrate | 0.024 g/mL | 0.024 g/mL | 0.024 g/mL | 0.024 g/mL |
| | | | | |
| BSA | $1.0 \times 10^{-6}$ g/mL | $1.0 \times 10^{-6}$ g/mL | $1.0 \times 10^{-6}$ g/mL | $1.0 \times 10^{-6}$ g/mL |
| $CaCl_2$ dihydrate | 0.0018 g/mL | 0.0018 g/mL | 0.0018 g/mL | 0.0018 g/mL |
| plasmin | 0 | 9.7 $\mu$g/mL | 11.4 $\mu$g/mL | 13.6 $\mu$g/mL |

Test result was determined for each of the assays as shown in **Table 10.** Clot reading % results were determined, as shown in **Table 11.** Reading differences are shown in **Table 12.** Mean Clot reading% was 95%. The reading difference absolute number was around 2 sec.

**Table 10. Test Result Mean Value Based on Three Replicates**

| | Samples | | | | |
|---|---|---|---|---|---|
| Added Plasmin Conc $\mu$g/mL | 2012 | 2365 | 2275 | 2447 | 2450 |
| 0.0 | 31.3 | 31.3 | 30.7 | 32.3 | 32.3 |
| 9.7 | 29.3 | 31.7 | 30.0 | 31.0 | 31.0 |
| 11.4 | 32.0 | 29.0 | 29.7 | 30.3 | 30.3 |
| 13.6 | 27.0 | 27.3 | 27.3 | 29.7 | 32.7 |

**Table 11. Clot Reading %**

| Added Plasmin Conc μg/mL | Samples | | | | |
|---|---|---|---|---|---|
| | 2012 | 2365 | 2275 | 2447 | 2450 |
| 0.0 | 100.0% | 100.0% | 100.0% | 100.0% | 100.0% |
| 9.7 | 93.6% | 101.1% | 97.8% | 95.9% | 95.9% |
| 11.4 | 102.1% | 92.6% | 96.7% | 93.8% | 93.8% |
| 13.6 | 86.2% | 87.2% | 89.1% | 91.8% | 101.0% |
| 17.0 | 200.0% | 200.0% | 200.0% | 200.0% | 200.0% |

**Table 12. Reading Difference (Absolute Number) with Plasmin versus Without Plasmin**

| Added Plasmin Conc μg/mL | Samples | | | | |
|---|---|---|---|---|---|
| | 2012 | 2365 | 2275 | 2447 | 2450 |
| 9.7 | 2.0 | 0.3 | 0.7 | 1.3 | 1.3 |
| 11.4 | 0.7 | 2.3 | 1.0 | 2.0 | 2.0 |
| 13.6 | 4.3 | 4.0 | 3.3 | 2.7 | 0.3 |

### *Example 4 - Reagent Including Silica/Tissue Factor*

Normal healthy patient blood samples were used for screening tests. Each blood sample was tested with cuvettes containing no plasmin in parallel with cuvettes containing five levels of plasmin.

The assays were performed according to a method described herein, wherein blood samples from 5 donors were tested on a GEM Hemochron®100 instrument to obtain test results. The composition of the reagents used in the assay are described in **Table 13.** Test results are shown in **Table 14.**

**Table 13. Composition of the Reagents in Solution**

| Component | Reagent | | | | |
|---|---|---|---|---|---|
| | Reagent 1 | Reagent 2 | Reagent 3 | Reagent 4 | Reagent 5 |
| HEPES | 0.004 g/mL | 0.004 g/mL | 0.004 g/mL | 0.004 g/mL | 0.004 g/mL |
| NaOH | 0.0084 g/mL | 0.0084 g/mL | 0.0084 g/mL | 0.0084 g/mL | 0.0084 g/mL |
| Fumed silica | 0.6% (v/v) | 0.6% (v/v) | 0.6% (v/v) | 0.6% (v/v) | 0.6% (v/v) |
| RTF | 4.0% (v/v) | 4.0% (v/v) | 4.0% (v/v) | 4.0% (v/v) | 4.0% (v/v) |
| Trehalose dihydrate | 0.024 g/mL | 0.024 g/mL | 0.024 g/mL | 0.024 g/mL | 0.024 g/mL |
| $CaCl_2$ dihydrate | 0.0018 g/mL | 0.0018 g/mL | 0.0018 g/mL | 0.0018 g/mL | 0.0018 g/mL |
| plasmin | 0 | 9.7 μg/mL | 11.4 μg/mL | 13.6 μg/mL | 17.0 μg/mL |

Test results are shown in **Table 14.** The results showed poor precision, with mean coefficient of variation (CV%) greater than 20%. Mean of Clot reading% was not calculated due to widespread data. Without wishing to be bound by theory, it is believed that selection of other components in the formulation impact silica colloid and cause aggregation.

**Table 14. Test Result Mean Value Based on Three Replicates**

| Added Plasmin Conc μg/mL | Samples | | | | |
|---|---|---|---|---|---|
| | 2170 | 2085 | 1073 | 2345 | 2442 |
| 0.0 | 112.7 | 113.7 | 111.3 | 96.0 | 104.0 |
| 9.7 | 47.0 | 92.7 | 79.3 | 108.7 | 55.3 |
| 11.4 | 146.0 | 147.3 | 145.5 | 114.3 | 168.0 |

(continued)

| Added Plasmin Conc µg/mL | Samples | | | | |
|---|---|---|---|---|---|
| | 2170 | 2085 | 1073 | 2345 | 2442 |
| 13.6 | ** | ** | ** | ** | ** |
| 17.0 | ** | ** | ** | ** | ** |
| ** Indicates out-of-range-high reading | | | | | |

### Analysis of Results from Examples 1-4

The tests described in Examples 1-4 demonstrated that the compositions and methods described herein can be used to test for hemostasis disorders such as hyperfibrinolysis and fibrinolytic shutdown. The results for mean of clot reading percentage and mean reading difference (absolute number) determined in each of Examples 1-4 (summarized in Table 14) showed that the reagents used in Example 1 (including phospholipid, silica, and tissue factor) provided the lowest mean of clot reading percentage (47%) and the largest mean reading difference (42 sec). These results demonstrated that the reagents used in Example 1 were preferable to those in Examples 2-4. Specifically, a lower mean of clot reading percentage and a higher mean reading difference are preferable because they may indicate higher sensitivity and specificity of the assay for diagnosing hyperfibrinolysis or shutdown.

As discussed above, the larger the gap between healthy and hyperfibrinolytic patients, the likelier that T/C of healthy people and T/C of hyperfibrinolytic patients can be clearly divided statistically in clinical test with fewer false positive or false negative results. The calculation of T-C is an alternative presentation of this data, which presents absolute difference while T/C is for relative difference. Again, it is preferable to maximize the absolute difference to achieve improved test performance.

Accordingly, the results demonstrated that the reagent with phospholipid, silica, and tissue factor is superior to tissue factor (without phospholipid or silica), phospholipid and tissue factor (without silica), or silica and tissue factor (without phospholipid).

**Table 14**

| | Example 1 (Phospholipid/Silica/ Tissue Factor) | Example 2 (Tissue Factor) | Example 3 (Phospholipid/ Tissue Factor) | Example 4 (Silica/Tissue Factor) |
|---|---|---|---|---|
| Mean of Clot Reading Percentage | 47 | 74 | 95 | ** |
| Mean Reading Difference (absolute value in sec) | 42 | 13 | 2 | ** |
| ** Unable to obtain usable results due to poor precision. | | | | |

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims. Variations, changes, and substitutions will now occur to those skilled in the art without departing from the invention. It may be understood that various alternatives to the embodiment of the invention described herein may be employed in practicing the invention. It is intended that the following claims define the scope of the invention and that methods and structures within the scope of these claims and their equivalents be covered thereby.

### Claims

1. A composition for use in a hemostasis blood test, the composition comprising:

   (a) a phospholipid component;
   (b) a surface activator;

(c) a blood coagulation initiator; and
(d) plasmin or a precursor thereof.

2. The composition of claim 1, further comprising one or more of:

(e) a buffering agent;
(f) a stabilizing agent;
(g) a protein concentration reference standard; and
(h) $CaCl_2$.

3. The composition of claim 1 or 2, wherein the phospholipid component comprises a plurality of phospholipids present in a concentration of about 0.001 mg/mL to about 1.0 mg/mL.

4. The composition of any one of claims 1-3, wherein the surface activator is present in a concentration of about 0.01% to about 1.0%.

5. The composition of any one of claims 1-4, wherein the blood coagulation initiator is tissue factor and the tissue factor is present in a concentration of about 0.000001 mg/mL to about 0.001 mg/mL.

6. The composition of any one of claims 1-5, wherein the plasmin or precursor thereof is present in a concentration of about 2.0 $\mu$g/mL to about 24 $\mu$g/mL.

7. The composition of any one of claims 1-6, wherein the surface activator comprises silica particles.

8. A method of detecting a hemostasis disorder in a subject, the method comprising:

(a) obtaining a blood sample from the subject;
(b) contacting at least a portion of the blood sample to an assay reagent, wherein the assay reagent comprises the composition of any one of claims 1-7, thereby generating an assay sample;
(c) performing a blood coagulation assay on the assay sample and measuring a time parameter of the blood coagulation assay; and
(d) comparing the time parameter to a reference clotting time, wherein a higher time parameter compared to the reference clotting time indicates that the subject has a hemostasis disorder.

9. The method of claim 8, wherein the higher time parameter is at least one or more seconds higher than the reference clotting time.

10. The method of claim 8 or 9, wherein a lower time parameter compared to the reference clotting time indicates that the subject does not have hyperfibrinolysis or fibrinolytic shut down.

11. The method of any one of claims 8-10, wherein the assay reagent is disposed in a sample holder.

12. The method of any one of claims 8-11, wherein the assay reagent is a dry reagent.

13. The method of any one of claims 8-12, wherein the blood coagulation assay comprises a microfluidic device-based assay.

14. The method of any one of claims 8-13, the method further comprising treating the subject, based on the subject being determined to have a hemostasis disorder.

15. A sample holder for use in a blood coagulation assay, the sample holder comprising an assay reagent comprising the composition of any one of claims 1-7.

16. The sample holder of claim 15, wherein the sample holder comprises a cartridge or a cuvette.

17. The sample holder of claim 15 or 16, wherein the assay reagent is provided in a dried form in the sample holder.

18. The sample holder of any one of claims 15-17, further comprising a control reagent comprising:

a. a phospholipid component;
b. a surface activator; and
c. a blood coagulation initiator.

19. The sample holder of any one of claims 15-18, wherein the blood coagulation assay detects a hemostasis disorder in a blood sample.

20. The sample holder of claim 19, wherein the blood sample is a whole blood sample.

FIG. 1

FIG. 2

# EUROPEAN SEARCH REPORT

**Application Number**

EP 25 17 8501

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/268483 A1 (GOLDENBERG NEIL A [US] ET AL) 30 October 2008 (2008-10-30) * paragraphs [0002], [0058], [0083]; claims 7,16,17 * | 1-20 | INV. G01N33/86 |
| A | CN 104 076 156 A (SYSMEX CORP) 1 October 2014 (2014-10-01) * paragraph [0020]; claims 1,6,8 * | 1-20 | |
| A | US 2020/208194 A1 (YAFFE MICHAEL B [US] ET AL) 2 July 2020 (2020-07-02) * paragraphs [0049], [0083]; claims 1,6,28; example 1 * | 1-20 | |
| A | JP 2022 123129 A (SYSMEX CORP) 23 August 2022 (2022-08-23) * paragraph [0020] - paragraph [0023] * | 1-20 | |

**TECHNICAL FIELDS SEARCHED (IPC)**

G01N

The present search report has been drawn up for all claims

| Place of search | Date of completion of the search | Examiner |
|---|---|---|
| The Hague | 2 October 2025 | Kosten, Jonas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 8501

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2025

| Patent document cited in search report | | | Publication date | Patent family member(s) | | Publication date |
|---|---|---|---|---|---|---|
| US 2008268483 | A1 | | 30-10-2008 | AU 2005289774 A1 | | 06-04-2006 |
| | | | | CA 2623142 A1 | | 06-04-2006 |
| | | | | EP 1802766 A2 | | 04-07-2007 |
| | | | | US 2008268483 A1 | | 30-10-2008 |
| | | | | WO 2006036744 A2 | | 06-04-2006 |
| CN 104076156 | A | | 01-10-2014 | CN 104076156 A | | 01-10-2014 |
| | | | | EP 2790024 A1 | | 15-10-2014 |
| | | | | JP 2014190954 A | | 06-10-2014 |
| | | | | US 2014295470 A1 | | 02-10-2014 |
| US 2020208194 | A1 | | 02-07-2020 | US 2020208194 A1 | | 02-07-2020 |
| | | | | US 2024360492 A1 | | 31-10-2024 |
| | | | | WO 2018209244 A1 | | 15-11-2018 |
| JP 2022123129 | A | | 23-08-2022 | CN 107340272 A | | 10-11-2017 |
| | | | | EP 3239713 A1 | | 01-11-2017 |
| | | | | JP 7101351 B2 | | 15-07-2022 |
| | | | | JP 7477117 B2 | | 01-05-2024 |
| | | | | JP 2017198594 A | | 02-11-2017 |
| | | | | JP 2022123129 A | | 23-08-2022 |
| | | | | US 2017315142 A1 | | 02-11-2017 |

EPO FORM P0459

For more details about this annex : see Official Journal of the European Patent Office, No. 12/82

## EP 4 653 871 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63651747
- US 10175225 B
- US 11242848 B
- US 11366093 B